# EUROPEAN PATENT SPECIFICATION

(11) **EP 3 927 408 B1**
(45) Date of publication and mention of the grant of the patent: **10.06.2026**
(21) Application number: 19916372.6
(22) Date of filing: 12.12.2019
(51) Int. Cl.: A61M 25/06, A61M 25/00, A61M 25/01

(54) **GUIDE EXTENSION CATHETER**
FÜHRUNGSVERLÄNGERUNGSKATHETER
CATHÉTER D'EXTENSION DE GUIDAGE

(30) Priority: 19.02.2019 US 201962807613 P
(43) Date of publication of application: 29.12.2021
(73) Proprietor: Teleflex Life Sciences LLC, Wilmington, DE 19808 (US)
(72) Inventor: BRENIZER, Joshua, Minneapolis, Minnesota 55369 (US)
(74) Representative: Marks & Clerk LLP
(86) International application number: PCT/US2019/065844
(87) International publication number: WO 2020/171878

(56) References cited:
- US-A- 4 402 685
- US-A- 5 324 262
- US-A1- 2003 004 537
- US-A1- 2003 144 657
- US-A1- 2003 144 657
- US-A1- 2016 346 515
- US-A1- 2017 028 170
- US-A1- 2017 028 170
- US-B2- 8 996 095

## Description

### CLAIM OF PRIORITY

Benefit of priority is hereby claimed to U.S. provisional patent application bearing serial no. 62/807,613, entitled "GUIDE EXTENSION CATHETER" and filed on February 19, 2019.

### TECHNICAL FIELD

The subject matter of this patent document relates to the field of medical devices. More particularly, but not by way of limitation, the subject matter relates to guide extension catheters for use with guide catheters.

### BACKGROUND

Interventional cardiology procedures often involve inserting guidewires or other instruments through catheters into coronary arteries that branch off from the aorta. In coronary artery disease, the coronary artery may be narrowed or occluded by atherosclerotic plaques or other lesions. These lesions may totally obstruct the lumen of the artery or may dramatically narrow the lumen of the artery. A narrowing is referred to as a stenosis. In order to diagnose and treat obstructive coronary artery disease it is commonly necessary to pass a guidewire or other instruments through and beyond the occlusion or stenosis of the coronary artery.

To treat a stenosis, a guide catheter can be inserted through the aorta and into or adjacent the ostium of the coronary artery. This is sometimes accomplished with the aid of the guidewire. The guide catheter is typically seated into or adjacent the opening or ostium of the artery to be treated and the guidewire or other instrument is passed through the lumen of the guide catheter and inserted into the artery beyond the occlusion or stenosis. Crossing tough lesions or tortuous anatomy can create enough backward force to dislodge the guide catheter from the ostium of the artery being treated. This can make it difficult or impossible for the interventional cardiologist to treat certain forms of coronary artery disease.

A coaxial guide catheter can be used in conjunction with a standard guide catheter to provide additional backup support. The coaxial guide catheter can be passed through the standard guide catheter until its distal end extends beyond the distal end of the standard guide catheter, thereby positioning the distal end of the coaxial guide catheter further within the branch artery harboring the stenosis. Coaxial guide catheters may thus be referred to as guide extension catheters.

While preexisting guide extension catheters may provide increased backup support, the structure of such catheters may interfere with the insertion of interventional devices and may be limited with respect to size. Accordingly, new devices and associated techniques capable of minimizing device interactions and accessing differently sized vessels are desired.

US2003/144657A1 proposes a catheter assembly that employs an outer catheter with a pre-formed distal end and an open lumen. An inner catheter having an open lumen and a pre-formed distal end is movably disposed within the outer catheter. Relative rotation and extension of the inner and outer catheters provides the distal end of the catheter assembly with an adjustable range of two- and three-dimensional shapes. The inner catheter can include sections of varying stiffness, such that extension of the inner catheter within the outer catheter modifies the shape of the outer catheter's pre-formed distal end.

US2017/028170A1 proposes a guide catheter extension device for use with a standard guide catheter. The guide catheter extension device is made up of a flexible, elongate extension catheter having a tapered tip portion at a distal end, an opening at a proximal end, and a body portion extending between the two. The extension catheter has a longitudinal slit extending from the distal tip portion toward the proximal opening. Methods of using the guide catheter extension device to aid in performing interventional cardiology procedures

### OVERVIEW

The present inventor recognizes that there is a need to provide guide extension catheters that are compatible with guide catheters for performing interventional procedures in challenging anatomy, e.g., narrow blood vessels harboring robust occlusions. The present inventor also recognizes that there is a need to reduce structural interactions between guide extension catheters and interventional devices inserted therethrough. The present inventor further recognizes that guide extension catheters having various diameters are also needed to perform interventional procedures in differently sized vessels. A guide extension catheter can be configured for delivery over a standard guide catheter to access discrete regions of coronary or peripheral vasculature and to facilitate accurate placement of interventional devices without interfering with the passage of such devices through the guide catheter. A guide extension catheter can further include distal tubing having a variable cross-sectional diameter.

Guide extension catheters and related methods are disclosed in this patent document. A guide extension catheter can comprise an elongate tube member (also referred to as guide extension tubing) and a push member (also referred to as a substantially rigid portion, push rod, or push wire). The push member, which may not have a lumen large enough to allow passage of interventional cardiology devices in some examples, can be eccentrically coupled relative to the tube member for slidably positioning the tube member around and partially beyond a distal end of a guide catheter into a vessel ostium of interest.

These and other embodiments and features of the present guide extension catheters and related methods will be set forth, at least in part, in the following Detailed Description. This Overview is intended to provide non-limiting embodiments of the present subject matter; it is not intended to provide an exclusive or exhaustive explanation of the disclosed embodiments. The Detailed Description below is included to provide further information about the present guide extension catheters and methods.

### BRIEF DESCRIPTION OF THE DRAWINGS

In the drawings, like numerals can be used to describe similar features and components throughout the several views. The drawings illustrate generally, by way of example, but not by way of limitation, various embodiments discussed in this patent document.
- FIG. **1**: illustrates a plan view of a guide catheter advanced through an aorta to an ostium of a coronary vessel.
- FIG. **2**: illustrates a plan view of a guide extension catheter, as constructed in accordance with at least one embodiment, used in conjunction with a guide catheter for the delivery of an interventional device into an occluded vessel for treatment.
- FIG. **3A**: illustrates a side view of a guide extension catheter, as constructed in accordance with at least one embodiment, showing an elongate tube member surrounding a guide catheter.
- FIG. **3B**: illustrates a side view of the guide extension catheter of FIG. 3A, showing a portion of the elongate tube member extended beyond a distal end of the guide catheter.
- FIG. **4**: illustrates a cross-sectional view of a push member, as constructed in accordance with at least one embodiment, surrounding a guide catheter and within an introducer sheath.
- FIG. **5**: illustrates a cross-sectional view of the push member of FIG. 4, as constructed in accordance with at least one embodiment, showing an elongate tube member surrounding the guide catheter.
- FIG. **6**: illustrates a perspective view of a guide extension catheter, as constructed in accordance with at least one embodiment.
- FIG. **7**: illustrates a perspective view of another guide extension catheter, as constructed in accordance with at least one embodiment.
- FIG. **8A**: illustrates a perspective view of another guide extension catheter surrounding a portion of a guide catheter, as constructed in accordance with at least one embodiment.
- FIG. **8B**: illustrates a cross-sectional view of a the guide extension catheter of FIG. 8A.
- FIG. **9**: illustrates a cross-sectional view of another guide extension catheter, as constructed in accordance with at least one embodiment.
- FIG. **10**: illustrates a perspective view of another guide extension catheter surrounding a portion of a guide catheter, showing a tubular push member defining a longitudinal slit.
- FIG. **11**: illustrates a perspective view of another guide extension catheter surrounding a portion of a guide catheter, showing a tubular push member configured to constrict.
- FIG. **12A**: illustrates a perspective view of another guide extension catheter surrounding a portion of a guide catheter, showing a push member configured to snap or clip ono the guide catheter.
- FIG. **12B**: illustrates a cross-sectional view of the guide extension catheter of FIG. 12A.
- FIG. **13A**: illustrates a perspective view of a reinforcement member included in a guide extension catheter in accordance with at least one embodiment.
- FIG. **13B**: illustrates a perspective view of another reinforcement member included in a guide extension catheter in accordance with at least one embodiment.

The drawings are not necessarily to scale. Certain features and components may be shown exaggerated in scale or in schematic form, and some details may not be shown in the interest of clarity and conciseness.

### DETAILED DESCRIPTION

This patent document discloses guide extension catheters to be placed over guide catheters for providing support and guidance in a vessel when percutaneously advancing interventional devices, such as balloon catheters, stents, or stent catheters. A guide extension catheter is configured to be passed over a guide catheter so that its distal end portion can be extended beyond a distal end of the guide catheter and into the desired vessel while its proximal or intermediate portions may remain around an outer surface of the guide catheter.

It is believed that the present guide extension catheters will find great utility by interventional cardiologists performing percutaneous transluminal coronary interventions. Although the remainder of this patent document generally discusses and illustrates such uses, it should be understood that the guide extension catheters can also be used for treating other non-coronary diseased vessels or other hollow structures (e.g., biliary tract, ureter, etc.) throughout a patient's body where interventional devices are or can be employed.

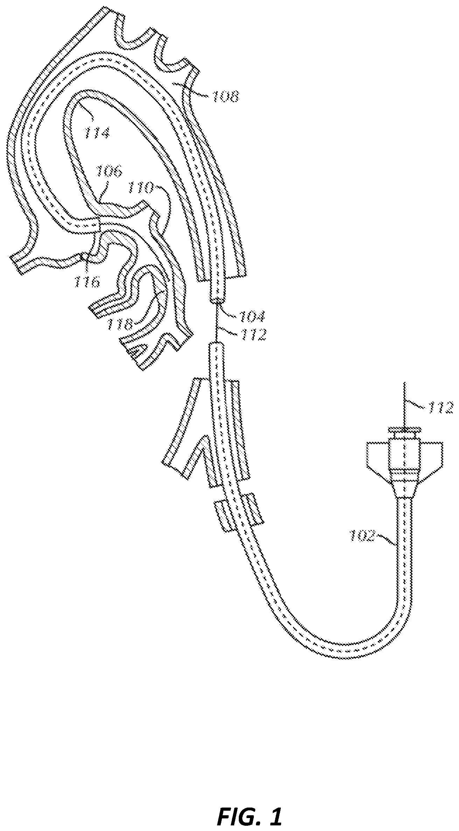

Minimally invasive cardiac interventions are utilized throughout the world and often include the use of a guidewire **112** and a guide catheter **102,** as illustrated in FIG. **1****.** The guidewire **112** can comprise an elongate, small-diameter member designed to navigate vessels to reach a diseased site or vessel segment of interest. Guidewires can come in various configurations, including solid steel or nitinol core wires and/or solid core wire wrapped in a smaller wire coil or braid, for example. The guide catheter **102** can comprise an elongate tube member defining a main lumen **104** along its length. The guide catheter **102** can be formed of polyurethane, for example, and can be shaped to facilitate its advancement to a coronary ostium **106** (or other region of interest within a patient's body). Any sized guide catheter **102,** such as a 6F, 7F, 8F guide catheter, where F is an abbreviation for the French catheter scale (a unit to measure catheter diameter (1F=⅓mm)), can be inserted at a femoral or radial artery and advanced through an aorta **108** to a position adjacent to the ostium **106** of a coronary artery **110.**

In a typical procedure, the guidewire **112** and guide catheter **102** can be advanced through the arch **114** of the aorta **108** to the ostium **106.** The guidewire **112** may then be advanced beyond the ostium **106** and into the coronary artery **110.** The diameter and rigidity of the guide catheter's distal end **116,** however, may not permit the device to be advanced beyond the ostium **106** and into the coronary artery **110.**

Maintaining the position of the guide catheter's distal end **116** at the ostium **106** can facilitate the guidewire **112** or other interventional device successfully reaching the diseased site (e.g., a stenotic lesion **118)** through its further distal advancement. With the guide catheter **102** in position, force can be applied to the guidewire's proximal end to push the guidewire **112** to and beyond the lesion **118,** and a treating catheter (optionally including a balloon or stent) can be passed over the guidewire **112** to treat the site. The application of force to the guidewire **112** or the treating catheter can sometimes cause the guide catheter **102** to dislodge from the ostium **106** of the coronary artery **110,** and, in such instances, the guidewire or treating catheter must be further distally advanced independently of the guide catheter's alignment and support to reach the lesion **118.** This can occur in the case of a tough stenotic lesion **118** or tortuous anatomy, where it is often difficult to pass the guidewire **112** or the treating catheter to and beyond the lesion. A heart's intrinsic beat can also cause the guide catheter's distal end **116** to lose its positioning or otherwise be shifted so that it no longer is positioned to align and support the guidewire **112** or the treating catheter into the portion of the coronary artery **110** including the lesion **118.**

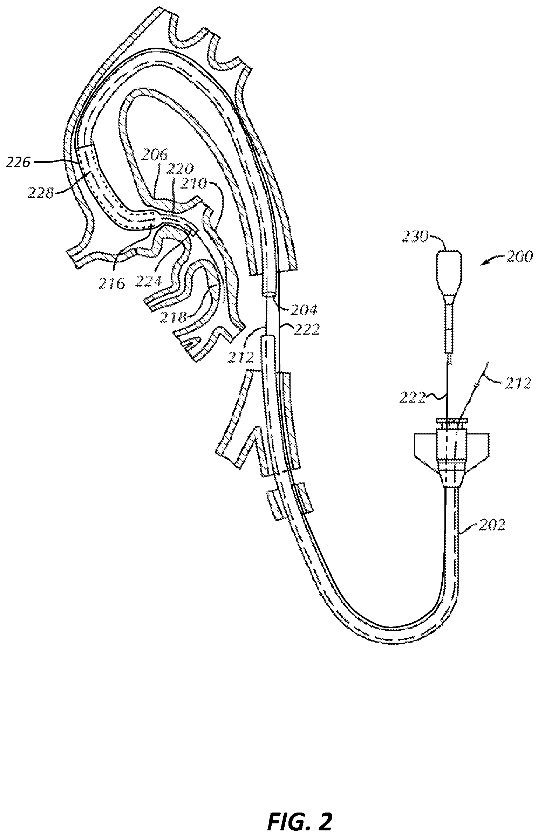

As illustrated in FIG. **2****,** the present guide extension catheter **200** can improve access to a coronary artery **210** and a stenotic lesion **218.** The guide extension catheter **200** can include a relatively flexible elongate tube member **220** and a push member **222** having a collective length that is greater than a length of a guide catheter **202** (e.g., 130cm-175cm, or greater). In embodiments, the combined length of the push member **222** and elongate tube member **220** can be limited such that upon full distal extension of the guide extension catheter **200,** the elongate tube member **220** does not fully extend beyond a distal end **216** of the guide catheter **202.** By sizing the guide extension catheter **200** in this manner, at least a proximal portion of the elongate tube member **220** may always surround a portion of the guide catheter **200,** thereby not interfering with the extension of interventional devices beyond the guide catheter's distal end **216.** In addition or alternatively, the guide extension catheter **200** may include one or more structures configured to limit its distal extension, such as a proximal, outwardly-fanning funnel having a cross-sectional diameter larger than that of the guide catheter **202** and/or an introducer sheath. An outer diameter of the tube member **220** can be sized to permit insertion of its distal end portion **224** into a coronary artery or its branches containing the lesion **218,** thereby providing alignment and support for an interventional device (e.g., a treating catheter) beyond the distal end **216** of the guide catheter **202** to the lesion and beyond. The extension of the tube member **220** into a smaller-sized artery or branch also serves to maintain the position of the guide catheter **202** at an artery's ostium **206** during an operation.

The operating physician can advance the distal end portion **224** of the tube member **220** over a guidewire **212** and the guide catheter **202** until it extends beyond the guide catheter's distal end **216** into the coronary artery **210** by applying a longitudinal force to the push member **222.** As shown, at least a distal portion of the tube member **220** may comprise a structure and/or material configured to constrict, such that all or a portion of the tube member **220** decreases in diameter after its extension beyond the guide catheter's distal end **216.** A proximal end portion **226** of the tube member **220** can remain around the guide catheter **202.** The physician can subsequently deliver a treating catheter over the guidewire **212,** through a main lumen **204** of the guide catheter **202** and a lumen **228** of the tube member **220** until the working portion of the treating catheter is located beyond the distal end portion **224** of the tube member. The operating physician can then treat the lesion **218** using standard techniques with added backup support on the guide catheter **202,** thereby providing an extra ability to push and advance the treating catheter.

In general, the lumen **228,** and hence the tube member **220,** can be sized and shaped to pass one or more interventional devices such as the guidewire **212,** guide catheter **202,** and treating catheter therethrough. The cross-sectional shape of the lumen **228** can be similar to the cross-sectional shape of the guide catheter's main lumen **204.** For instance, in some examples, the cross-sectional shape of the lumen **228** can be generally uniform along its length. In other examples, the cross-sectional diameter may vary along the length of the tube member **220.**

The outer diameter of the tube member **220** can assume minimum cross-sectional dimensions that allow the tube member **220** to coaxially slide over the guide catheter **202.** In other embodiments, the outer cross-sectional dimensions of the tube member **220** can be greater than the allowable minimum. For example, with a 6F guide catheter, the tube member **220** can have about a 7F, 8F, 9F or greater diameter, or any diameter therebetween. In some embodiments, a diameter of the lumen **228** of the tube member **220** may not be more than about one French size larger than an outer diameter of the guide catheter **202.** In one embodiment, the guide extension catheter **200** can be made in at least three sizes corresponding to the internal capacity of 8F, 7F, and 6F guide catheters that are commonly used in interventional cardiology procedures. The difference in size between the inner diameter of the tube member **220** and the outer diameter of the guide catheter **202** may vary. For instance, the gap in cross-sectional diameter between the outer diameter of the guide catheter and the inner diameter of the tube member **220** may be less than and/or about 0.0254 mm (0.001 in)., 0,0508 mm (0.002 in), 0.0762 (0.003 in), 0.127 mm (0,005 in), or any distance therebetween. In specific embodiments, the cross-sectional diameter gap may range from about 0,0508 to 0.0762 mm (0,002 to 0.003 in), or about 0.0508 mm to 0.0889 mm (0.002 to 0.0035 in.).

The diameter gap between an inner diameter of the tube member **220** and outer diameter of the guide catheter **202** may also be generally continuous along a substantial portion of the length or a majority of the length of the tube member **220** in some embodiments. In various embodiments, a guide catheter **202** with any diameter may be used. The length of the tube member **220** can be substantially less than the length of the guide catheter **202;** however, the tube member **220** can be designed with any length according to a desired application, such as about 6 to about 45 cm, about 10 to about 35 cm, about 14 to about 25 cm, or about 18 to about 20 cm.

The push member **222** can be attached to the proximal end portion **226** of the tube member **220** and can extend proximally from this attachment to a handle member **230** (also referred to as a manipulation member) accessible to an operating physician outside of a patient's body. The handle member **230** and push member **222** can allow the physician to position the tube member **220** between a first position, entirely surrounding the guide catheter **202,** and the illustrated second position, in which the tube member's distal end **224** extends beyond that of the guide catheter **202** and into the coronary artery **210.** The push member **222** can be rigid enough to allow the guide extension catheter **200** to be inserted over the guide catheter **202** upon receiving a pushing force from a physician via the handle member **230.** In some examples, the push member **222** can be more rigid along its longitudinal axis than the tube member **220,** and may comprise a rod, wire, or rail structure without a lumen through which interventional cardiology devices and the guide catheter **202** are insertable.

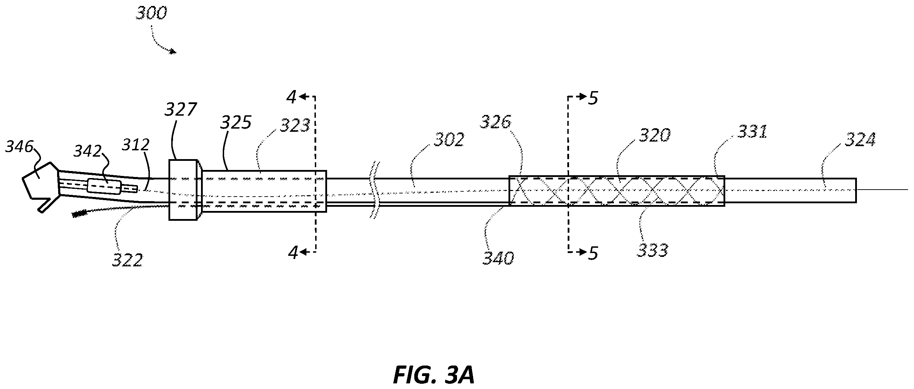

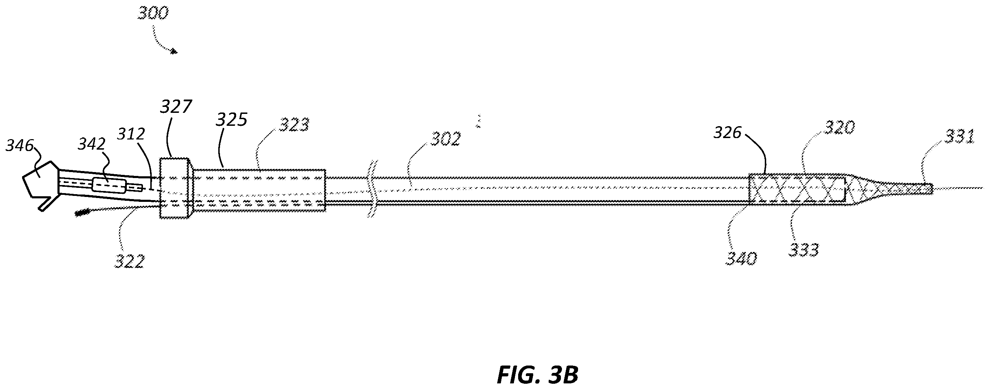

FIG. **3A** illustrates a side view of an example guide extension catheter **300** in accordance with embodiments of the present disclosure. As shown, a guidewire **312** may extend through the guide extension catheter **300,** which can include an elongate tube member **320** coupled at a proximal end **326** with a push member **322** at the push member's distal end **340.** The tube member **320** can surround a portion of the guide catheter **302,** and may be advanced distally over the guide catheter **302** until at least a distal portion of it surpasses the guide catheter's distal end **324.** A hemostatic valve **346,** which can be traversed using a preassembled kit, is coupled to a proximal end of the guide catheter **302.** An introducer sheath **323** is also included near the proximal end of both the guide catheter **302** and the push member **322.** The introducer sheath **323** may generally include an elongate shaft **325** and a tubular entry port **327** configured to receive and facilitate entry and removal of the guide extension catheter **300** into a patient's vasculature. The tube member **320,** or at least a distal portion **331** thereof, may include an elastic material or structure, such as the shape-memory braid **333** shown in FIG. **3****,** which may be comprised of nitinol in some examples.

The introducer sheath **323** may reduce lateral and axial movement of the guide catheter **302** and guide extension catheter **300** used during a vascular procedure relative to the blood vessel wall, thereby reducing or eliminating vessel spasm. The length of the introducer sheath **323** may vary depending on the depth of the targeted vessel relative to the skin, said depth ranging from about 1 cm to about 10 cm in some embodiments, or depths less or greater than this range. The introducer sheath **323** defines a lumen, the diameter of which may also vary, configured to receive various elongated vascular instruments. While the introducer sheath **323** shown in FIG. **3** is generally cylindrical, any cross-section may be used (and the cross-section can vary along the length), and the shape of the sheath may also vary, defining converging and/or asymmetrical tip portions, for example.

The size and configuration of the guidewire **312** may also vary. In some examples, a hollow guidewire defining a lumen may be used. In other embodiments, the guidewire **312** may be solid. The diameter of the guidewire may vary depending on the diameter of the vessel lumen and/or the diameter of the other instruments employed during the operation. Specific embodiments may include a guidewire having a diameter ranging from about a 0.254 mm (0.01 in.) to about 102 mm (0.04 in.), about 0,330 mm (0.013 in.) to about 0.965 mm (0.038 in.), 0.381 mm (0.015 in.) to about 0.508 mm (0.02 in.), or about 0.457mm (0.018in.).

The length of the guidewire may also vary, including ranging from about 30 cm to about 270 cm, about 30 cm to about 80 cm, about 30 cm to about 70 cm, about 35 cm to about 45 cm, or about 55 cm to about 65 cm in various embodiments. In some examples, the guidewire may define a tapered tip portion at the distal end, which may also be curved to avoid perforation of the vessel wall. The guidewire **312** may comprise stainless steel in some embodiments. It is to be appreciated that the dimensions in this paragraph, and in this document, are exemplary only, and any suitable dimensions may be used.

With the guidewire **312** and the guide catheter **302** positioned as desired, the tube member **320** of the guide extension catheter **300** can be backloaded from its distal end portion **331** onto a proximal end of the guide catheter **302.** As shown in FIG. **3B****,** a portion of the tube member **320** of the guide extension catheter **300** can then be advanced beyond a distal end **324** of the guide catheter **302** under fluoroscopy. When so arranged, portions of the tube member **320** can engage an ostium and extend within a portion of a coronary artery to help maintain the position of the guide catheter **302** as a treating catheter **342** is advanced.

The push member **322** can be rigid enough to urge the tube member **320** through the vasculature in response to receiving an axial force applied at a proximal end thereof, e.g., by a physician. The stiffness of the push member **322** may be uniform, or substantially uniform, along its length. In certain examples, the push member **322** can include a plurality of segments or portions having different stiffness and flexibility profiles to provide the guide extension catheter **300** with a desired combination of pushing force and vessel placement capabilities. In some embodiments, the push member **322** can be an elongated solid wire of constant or varying dimensions and can be made of a polymeric or metallic material, such as high tensile stainless steel (e.g., 304V, 304L or 316LV), mild steel, nickel-titanium allows, nickel-chromium-molybdenum alloys, nickel-copper alloys, nickel-tungsten alloys or tungsten alloys. The push member **322** can be coated with a hydrophilic, silicone or other friction-reducing material.

In some examples, the tube member **320** can be formed from an inner polymer layer, an outer polymer layer, and/or a reinforcement member (e.g., braid or coil) disposed between or adjacent to the polymer layers, for example as described in U.S. Patent Nos. 8,048,032, 8,142,413, RE45,760, RE 45,380, RE45,776, and RE46 116.

According to such examples, the inner polymer layer can be composed of, or coated with, silicone, polytetrafluoroethylene (PTFE) or another lubricious material to provide a slippery surface for received interventional devices. The outer polymer layer can include one or more flexible materials, such as polyurethane, polyethylene or polyolefin of sequentially diminishing durometers along the tube member's length, and it can be coated with a friction-reducing material (e.g., a hydrophilic material) to facilitate insertion and trackability through vasculature and a guide catheter. The reinforcing braid or coil, in embodiments featuring a braid or coil, can be formed of stainless steel or a platinum alloy, for example, and can extend between the polymer layers along at least a portion of the tube member's length.

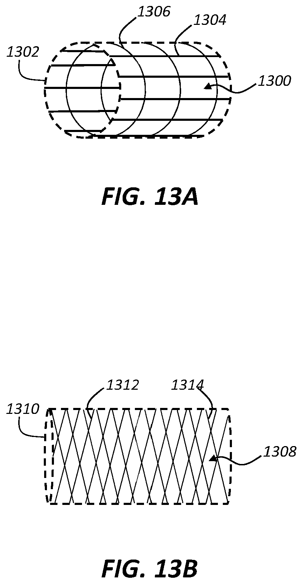

The optional reinforcement member disposed between the polymer layers of some elongate tube members **320** can be configured in multiple ways. For instance, the reinforcement member may lack a braid, coil or other distinct reinforcing structure, and may instead comprise one or more materials having greater stiffness than the remaining portions of the tube member **320.** In addition or alternatively, embodiments of the reinforcement member can include different reinforcing structures, e.g., a rigid sleeve, elongate member, and/or bars or strips of rigid or semi-rigid material, as shown in FIGS. **13A** and **13B****.** Additional components and/or materials configured to increase or decrease the rigidity of a portion of the tube member **320** are also contemplated. At least in part because the components of the reinforcement member may vary, methods of assembling the reinforcement member may also vary. For example, if the reinforcement member disposed between the polymer layers of the elongate member **320** includes a coil, various types of coils may be used, and in some examples, each coil can be coupled with other components of the tube member **320** in a distinct manner, which may depend on whether the cross-sectional diameter of the tube member is uniform or varied.

A proximal end portion **326** of the tube member **320** can be eccentrically coupled to a distal end portion **340** of the push member **322** at its periphery or circumference and can provide a smooth transition between the members in some examples. The arrangement or configuration of this coupling can vary. For example, the tube member **320** can include a side opening formed at a proximal end of its peripheral wall. In some examples, the push member **322** can be disposed within the opening. Inserting the push member **322** into the opening can result in a mechanical coupling between the members and additional or alternative bonds (e.g., adhesive bonds, thermal bonds, welds, brazes, etc.) can be utilized. The distal end portion **340** of the push member **322** can be flattened in some embodiments to provide a larger surface area to secure to the tube member **320.** In addition or alternatively, coupling mechanisms facilitated by a third component (e.g., a metal or polymer skived (slanted) collar or concave track) bonded between or integrated with the proximal end portion **326** of the tube member **320** or the distal end portion **340** of the push member **322** are also contemplated, for example as described in U.S. Patent Application No. 15/581,176.

Metallic or polymeric structures forming the third component can become less stiff and more flexible in a proximal-to-distal direction, for instance, to provide a gradual flexibility transition between the more rigid push member **322** and the more flexible tube member **320.**

Markers on the push member **322** or the tube member **320** can allow an operating physician to identify positioning of the guide extension catheter's components relative to patient anatomy, the guide catheter **302,** and any interventional devices used during a procedure. For example, one or more depth markers can be printed on an outer surface of the push member **322** and can be positioned at predetermined lengths relative to a distal end of the tube member **320.** One or more radiopaque marker bands can be positioned on the tube member **320.** The marker bands can be composed of tungsten, platinum or an alloy thereof and can have a metallic band structure. Alternatively, for space conservation reasons, the marker bands can be formed by impregnating portions of the tube member **320** with a radiopaque filler material, such as barium sulfate, bismuth trioxide, bismuth carbonate, powdered tungsten, powdered tantalum or the like. A first marker band can be positioned slightly distal to a fully-round entrance of the tube member **320** and a second marker band can be positioned near the tube member's distal end, for example.

FIG. **3B** illustrates another side view of the example guide extension catheter **300** shown in FIG. **3A****,** after distal extension of the tube member **320** via the push member **322.** As shown, at least a distal portion **331** of the tube member **320** may be configured to shrink or constrict when no longer structurally bound from the interior by the guide catheter **302.** Constriction of the tube member **320** may allow it to access narrow vessels without interfering with the passage of interventional devices through the guide catheter **302.** Additional examples of the tube member **320** may not exhibit a variable cross-sectional diameter, such that even when extended beyond the distal end of the guide catheter **302,** the tube member **320** may retain a generally constant or substantially constant diameter.

The decrease in cross-sectional diameter exhibited by examples of the tube member **320** configured to constrict may vary. For example, the diameter of the tube member **320** may decrease until its inner diameter matches or approximately matches the inner diameter of the guide catheter **302.** In some embodiments, such as the one depicted in FIG. **3B****,** the diameter may decrease until the inner diameter of the tube member **320** is smaller than the inner diameter of the guide catheter **302.** In various examples, the decreased inner diameter of the tube member **320** may be greater than 8F, or approximately 8F, 7F, 6F, 5F or less, or any diameter therebetween. The tube member **320** can be relatively flexible or elastic, such that its cross-sectional diameter increases again upon retraction over the guide catheter **302,** or upon receipt and passage of one or more interventional devices therethrough.

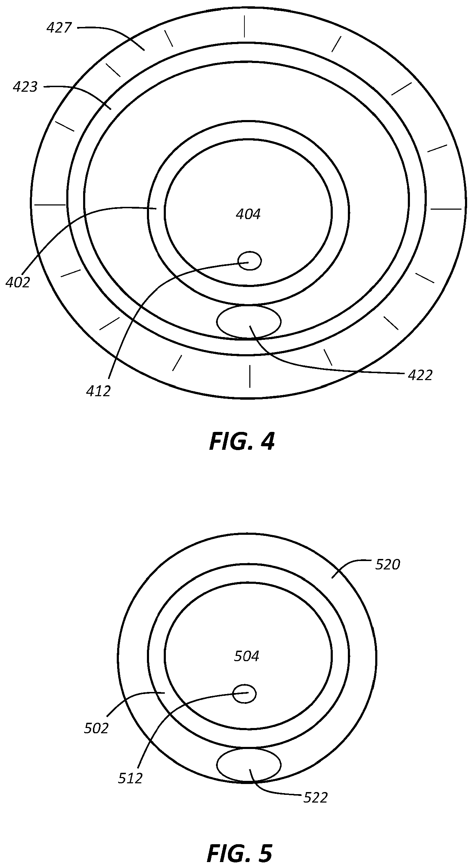

FIG. **4** illustrates a cross-sectional view of an example push member **422** near its proximal end portion, such as along line **4-4** of FIG. **3A****,** within an introducer sheath **423** defining a proximal entry port **427.** The push member **422** lies radially between the sheath **423** and a guide catheter **402,** which defines a lumen **404** containing a guidewire **412.** The push member **422** defines an oval cross-section in this particular example, but the cross-sectional shape and dimensions of the push member **422** may vary. For example, the push member **422** comprise an arcuate or flat, sheet-like cross-sectional shape, and rectangular, irregular, oval, oblong cross-sectional shapes are also within the scope of this disclosure. In operation, the guidewire **412,** introducer sheath **423,** and guide catheter **402** may all be introduced into a patient's vasculature prior to insertion of the guide extension catheter comprised of push member **422.**

FIG. **5** illustrates a cross-sectional view of a distal end portion of an example push member **522,** such as along line **5-5** of FIG. **3A****.** The push member **522** is coupled at a distal end with an elongate tube member **520,** which surrounds a guide catheter **502,** an inner lumen **504** of which includes a guidewire **512.** The distal end of the push member **522,** upon receiving an axial or longitudinal force by an operating physician, may contact and push the tube member **520** over the guide catheter **502.** The relative thickness of each component shown in FIG. **5** may vary. For example, the tube member **520** may define a cross-sectional thickness that is greater than, less than, or generally equal to the thickness of the guide catheter **502.**

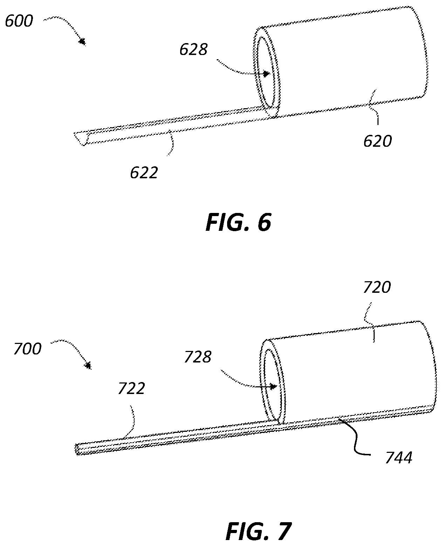

FIG. **6** illustrates a perspective view of a portion of a guide extension catheter **600** comprised of an elongate tube member **620** and a push member **622.** The elongate tube member **620** can be generally cylindrical and may define a lumen **628** configured to receive a guide catheter. The configuration of the push member **622** may vary. In the example shown, the push member **622** defines an arcuate, concave track configured to act as a rail for a guide catheter, thereby facilitating relative movement between the guide catheter, the push member **622,** and the elongate tube member **620.** The arc length, flexibility and degree of curvature defined by the push member **622** can vary (as further described below), and may depend on the circumference of the guide catheter used with the guide extension catheter **600.** For example, the arc length of the push member **622** may be greater to accommodate larger guide catheters, and vice versa.

FIG. **7** illustrates a perspective view of another guide extension catheter **700** comprised of an elongate tube member **720** defining a lumen **728** and attached to a push member **722.** In this example, the push member **722** defines a cylindrical or rod-like shape, and along with the tube member **720,** defines a longitudinal groove **744.** The groove **744** may extend along the entire length of the tube member **720** and push member **722,** or only a portion thereof. The groove **744** can receive and guide one or more additional interventional devices, e.g., guidewires, to the vessel target site. In some examples, the groove **744** may define a relatively smooth, concave surface, or straight opposing surfaces that meet at a point. Embodiments may include 2 or more grooves, for example on opposite sides of the guide extension catheter **700.** The grooves **744** are not limited to any particular embodiment, and may be included in guide extension catheter **300** and/or **600,** for instance.

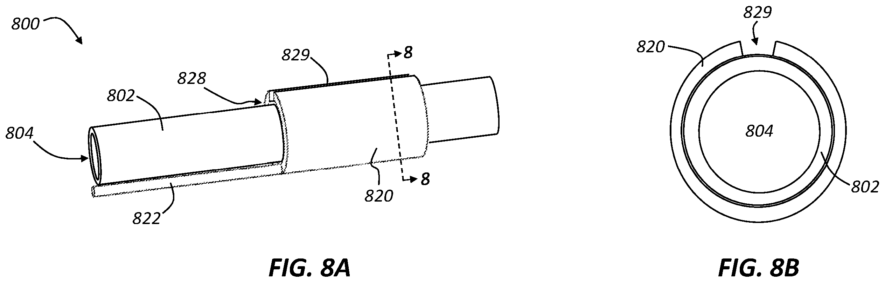

FIG. **8A** illustrates a perspective view of another guide extension catheter **800** comprised of an elongate tube member **820** defining a lumen **828** and attached to a push member **822.** A guide catheter **802** defining an inner lumen **804** is slidably coupled within the elongate tube member **820,** extending through the tube member's lumen **828.** The tube member **820** includes a longitudinal slit **829** that extends along its length. The slit **829** can have non-overlapping ends and can be formed to be resiliently closed, such that it can be forcibly peeled open to receive and/or remove the guide catheter **802.** Embodiments may involve urging a proximal end of the guide catheter **802** into the elongate tube member **820** before, during and/or after sliding the elongate tube member **820** distally along the guide catheter **802,** toward a targeted vessel site. After decoupling the guide catheter **802** and elongate tube member **820,** the tube member **820** may constrict, such that the longitudinal slit **829** closes or substantially closes, thereby narrowing the cross-sectional diameter of the tube member **820.** In other embodiments, the slit **829** may not constrict upon decoupling, instead temporarily expanding only during insertion or removal of the guide catheter **802.**

FIG. **8B** illustrates a cross-sectional view of the guide catheter **800,** taken along line **8-8** of FIG. **8A****.** While the longitudinal slit **829** is positioned above the guide catheter **802** in the configuration shown, the relative position of the slit **829** may vary. For example, the slit **829** may be positioned anywhere along the circumference of the elongate tube member **820,** extending below or alongside the guide catheter **802,** for instance. The width of the slit **829** may also vary. In embodiments, the width of the slit **829** in its unbiased, i.e., most narrow, configuration may be about 5%, 10%, 20%, 30%, 40%, 50% or more of the width of the guide catheter **802.** The width of the slit **829** may depend on the diameter of the guide catheter **802** and/or the flexibility of the elongate tube member **802.** For example, the width of the slit **829** may be smaller for a tube member **820** having greater flexibility, and vice versa.

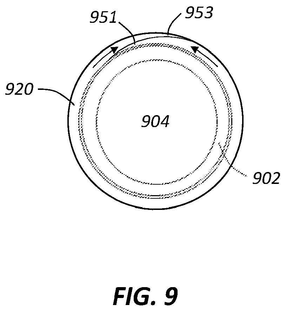

FIG. **9** illustrates a cross-sectional view of another elongate tube member **920** surrounding a guide catheter **902,** which defines an inner lumen **904.** The tube member **920** is biased to constrict around an outer surface of the guide catheter **902** via two overlapping ends **951, 953** configured to converge radially in the direction of the arrows. Upon extension of a portion of the tube member **920** beyond a distal end of the guide catheter **902,** the ends **951, 953** may continue to slide past each other, further constricting the tube member **920** until its diameter is approximately equal to or less than the inner diameter of the guide catheter **902.**

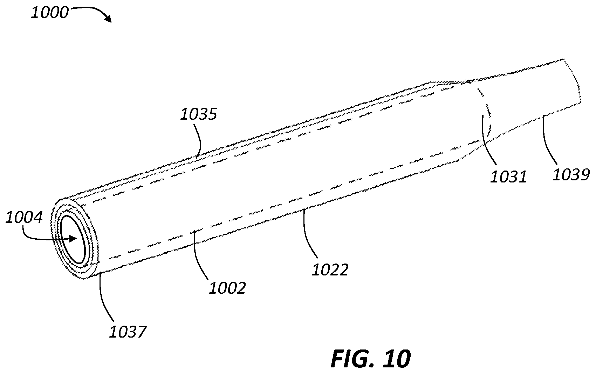

FIG. **10** illustrates a portion of a guide extension catheter **1000** surrounding a distal portion of a guide catheter **1002,** which defines a lumen **1004.** In this embodiment, the push member **1022** comprises an elongate tube defining a longitudinal slit **1035.** Like the slit **829** illustrated in FIGS. **8A/8B****,** the slit **1035** can be defined by non-overlapping ends of the push member **1022,** and can be formed to be resiliently closed, such that the slit **1035** can be forcibly peeled open to receive and/or remove the guide catheter **1002.** The push member **1022** may be configured to constrict upon its extension beyond the guide catheter's distal end **1031.** The entire length of the push member **1022** may be configured to constrict, or only a portion thereof. In some embodiments, only a distal portion **1039** may be configured to constrict, while a proximal portion **1037** may generally retain an approximately constant diameter. In such embodiments, the slit **1035** may be confined to the distal portion **1039** of the push member.

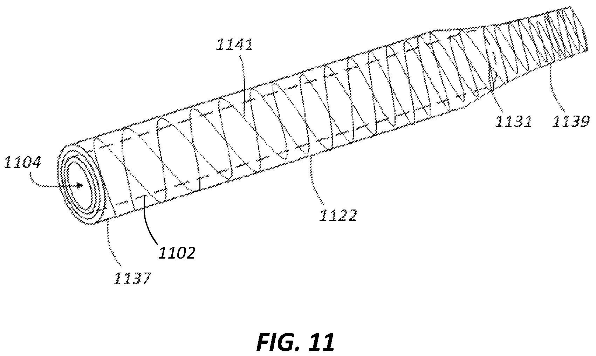

FIG. **11** illustrates a portion of another guide extension catheter **1100** surrounding a distal portion of a guide catheter **1102,** which defines a lumen **1104.** The push member **1122** again comprises an elongate tube configured to encompass and extend at least partially beyond a distal end **1131** of the guide catheter **1102.** The push member **1122,** or at least a distal portion **1139** thereof, may include an elastic material or structure configured to constrict, such as a shape-memory braid **1141,** which may be comprised of nitinol. In some examples, the braid **1141** (or other constricting structure/material) may be confined to the distal portion **1139,** such that only the distal portion is configured to constrict, e.g., upon extension beyond the guide catheter's distal end **1131.** The length of the push member **1122** configured to constrict may vary, for example such that the constrictable portion spans from a distal end of the push member **1122** and constitutes approximately less than or equal to 5%, 10%, 20%, 30%, 40%, 50%, 60%, 70%, 80%, 90%, or 100% of the length of the push member **1122,** or any length therebetween.

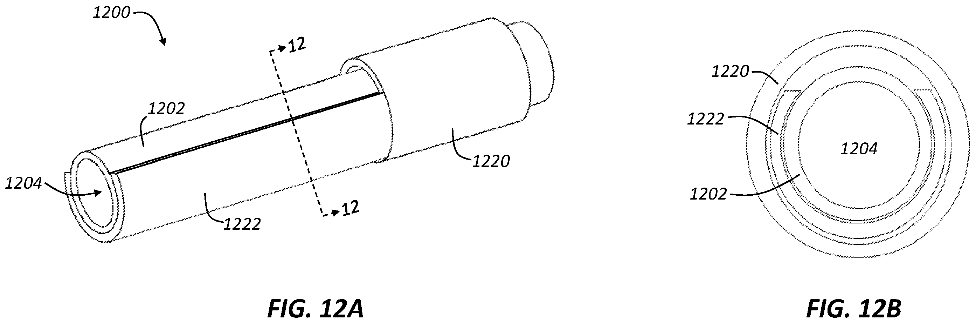

FIG. **12A** illustrates a perspective view of another guide extension catheter **1200** comprised of an elongate tube member **1220** attached to a push member **1222.** A portion of a guide catheter **1202** is shown extended through the elongate tube member **1220.** In this embodiment, the push member **1222** comprises a concave track configured to couple with the guide catheter **1202** via a clipping or snapping mechanism. By reversibly coupling the push member **1222** with the guide catheter **1202** in this manner, the push member **1222** (and elongate tube member **1220)** can be slidably guided to a vascular target site by the guide catheter **1202,** thereby reducing the likelihood of the guide extension catheter **1200** buckling or twisting, or even perforating a vessel wall, upon receiving an axial force applied by an operating physician. As shown, the push member **1222** may extend around greater than half the circumference of the guide catheter **1202.** The push member **1222** may comprise a relatively flexible, elastic or resilient material configured to bend upon coupling the guide catheter **1202** therewith. The push member **1222** and/or guide catheter **1202** may comprise a friction-reducing material, thereby allowing the two components to slide past each other during insertion of the guide extension catheter **1200.**

FIG. **12B** illustrates a cross-sectional view of the guide extension catheter **1200** taken along line **12-12** of FIG. **12A****,** showing the lumen **1204** defined by the guide catheter **1202,** which is partially surrounded by the push member **1222** and completely surrounded at a distal portion by the elongate tube member **1220.** In embodiments, the degree of enclosure defined by push member **1222** around the circumference of the guide catheter **1202** may vary, for example ranging from less than or equal to about 50%, 60%, 70%, 80%, 90%, or greater, or any degree therebetween.

Additional examples can include a push member having a shape that is approximately rectangular or flat, or a shape that changes along the length of the push member. The push member can also include multiple surfaces each having a distinct shape or curvature, for example as described in U.S. Patent Application No. 15/581,176. In some embodiments, the push member can be an elongated solid wire of constant or varying dimensions and can be made of a polymeric or metallic material, such as high tensile stainless steel (e.g., 304V, 304L or 316LV), mild steel, nickel-titanium allows, nickel-chromium-molybdenum alloys, nickel-copper alloys, nickel-tungsten alloys or tungsten alloys. The push member can be coated with a hydrophilic, silicone or other friction-reducing material. The stiffness of the push member may be uniform, or substantially uniform, along its length, or may define areas having variable stiffness along its length. For example, the push member may be more flexible near its distal end than its proximal end.

FIG. **13A** illustrates an example reinforcement member **1300,** which may be included in some embodiments to increase the stiffness of the elongate tube member **1302** of a guide extension catheter (only a portion of which is shown). As described above, the reinforcement member **1300** may be sandwiched between two polymer layers constituting the elongate tube member **1302.** The reinforcement member **1300** can include a plurality of longitudinal bars or strips **1304,** which may be interlaced with one or more cross-bars or strips **1306.** The strips **1304, 1306** may be arranged perpendicularly, or substantially perpendicularly, with respect to each other, or they may be diagonally arranged. In some examples, only the longitudinal or the cross strips may be included. The reinforcement member **1300** can extend around the entire perimeter of the elongate tube member **1302,** or only a portion thereof.

FIG. **13B** illustrates another example reinforcement member **1308** included with an elongate tube member **1310** (only a portion of which is shown). In this example, the reinforcement member **1308** can be comprised of spiraling bars or strips **1312, 1314,** which may criss-cross. In embodiments, strips in only one spiral direction, i.e., **1312 or 1314,** may be included. Any suitable angle or combination of angles of the spiral with respect to the longitudinal axis of the tube may be used. Like reinforcement member **1300,** reinforcement member **1308** can be sandwiched between individual layers constituting the elongate tube member **1310.** The particular configuration of the reinforcement member, its location and/or length may vary in different embodiments of the guide extension catheters disclosed herein, which are not confined to examples including reinforcement members, or specific embodiments thereof. The materials constituting the reinforcement member may also vary. In examples, the reinforcement member can include stainless steel, a platinum alloy, and/or one or more polymers, for instance.

### Examples:

The above Detailed Description is intended to be illustrative and not restrictive. The above-described embodiments (or one or more features or components thereof) can be used in varying combinations with each other unless clearly stated to the contrary. Other embodiments can be used, such as by one of ordinary skill in the art upon reviewing the above Detailed Description. Also, various features or components have been grouped together to streamline the disclosure. This should not be interpreted as intending that an unclaimed disclosed feature is essential to any claim. Rather, inventive subject matter can lie in less than all features of a particular disclosed embodiment.

### Closing Notes:

The above Detailed Description includes references to the accompanying drawings, which form a part of the Detailed Description. The Detailed Description should be read with reference to the drawings. The drawings show, by way of illustration, specific embodiments in which the present guide extension catheters and related methods can be practiced. These embodiments are also referred to herein as "examples."

Certain terms are used throughout this patent document to refer to particular features or components. As one skilled in the art will appreciate, different people may refer to the same feature or component by different names. This patent document does not intend to distinguish between components or features that differ in name but not in function. For the following defined terms, certain definitions shall be applied unless a different definition is given elsewhere in this patent document. The terms "a," "an," and "the" are used to include one or more than one, independent of any other instances or usages of "at least one" or "one or more." The term "or" is used to refer to a nonexclusive or, such that "A or B" includes "A but not B," "B but not A," and "A and B." All numeric values are assumed to be modified by the term "about," whether or not explicitly indicated. The term "about" refers to a range of numbers that one of skill in the art considers equivalent to the recited value (i.e., having the same function or result). In many instances, the term "about" can include numbers that are rounded to the nearest significant figure. The recitation of numerical ranges by endpoints includes all numbers and sub-ranges within and bounding that range (e.g., 1 to 4 includes 1, 1.5, 1.75, 2, 2.3, 2.6, 2.9, etc. and 1 to 1.5, 1 to 2, 1 to 3, 2 to 3.5, 2 to 4, 3 to 4, etc.). The terms "patient" and "subject" are intended to include mammals, such as for human or veterinary applications. The terms "distal" and "proximal" are used to refer to a position or direction relative to an operating physician. "Distal" and "distally" refer to a position that is distant from, or in a direction away from, the physician. "Proximal" and "proximally" refer to a position that is near, or in a direction toward, the physician. And the term "interventional device(s)" is used to include, but is not limited to, balloon catheters, stents and stent catheters.

The scope of the present guide extension catheters should be determined with reference to the appended claims. In the appended claims, the terms "including" and "in which" are used as the plain-English equivalents of the respective terms "comprising" and "wherein." Also, in the following claims, the terms "including" and "comprising" are open-ended; that is, a device or method that includes features or components in addition to those listed after such a term in a claim are still deemed to fall within the scope of that claim. Moreover, in the following claims, the terms "first," "second" and "third," etc. are used merely as labels, and are not intended to impose numerical requirements on their objects.

The Abstract is provided to allow the reader to quickly ascertain the nature of the technical disclosure. It is submitted with the understanding that it will not be used to interpret or limit the scope or meaning of the claims.

## Claims

1. A guide extension catheter (200) for use with a predefined length guide catheter (202), the guide extension catheter comprising:
an elongate tube member (220) defining a lumen (228) having a cross-sectional inner diameter through which the guide catheter is insertable; and
a push member (222) that is rigid enough to allow the elongate tube member to be urged over the guide catheter, the push member being proximal of, operably connected to, and more rigid along a longitudinal axis than the elongate tube member, the push member having a maximal cross-sectional dimension that is smaller than a cross-sectional outer diameter of the elongate tube member and having a length such that when combined with a length of the elongate tube member forming a guide extension catheter length along the longitudinal axis that is longer than the guide catheter.

2. The guide extension catheter of claim 1, further comprising an introducer sheath (323) defining a lumen having a cross-sectional inner diameter through which the elongate tube member is insertable.

3. The guide extension catheter of any one of claims 1 or 2, wherein the elongate tube member includes a longitudinal slit (829) along its length, the longitudinal slit biased toward a closed position.

4. The guide extension catheter of any one of claims 1 to 3, wherein the cross-sectional inner diameter of the elongate tube member is configured to adjust in response to a force applied thereto.

5. The guide extension catheter of claim 4, wherein the elongate tube member comprises a shape memory braid (333).

6. The guide extension catheter of claim 5, wherein the shape memory braid comprises nitinol.

7. The guide extension catheter of claim 4, wherein the cross-sectional inner diameter of the elongate tube member is configured to decrease until the cross-sectional inner diameter is approximately equal to a cross-sectional outer diameter of the guide catheter.

8. The guide extension catheter of any one of claims 1 to 7, wherein the push member comprises a rod or a wire.

9. The guide extension catheter of any one of claims 1 to 8, wherein the push member comprises a longitudinal groove (744) along its length.

10. The guide extension catheter of any one of claims 1 to 9, wherein the elongate tube member comprises an inner polymer layer and an outer polymer layer.

11. The guide extension catheter of any one of claims 1 to 10, wherein the elongate tube member, when its distal end is extended distally of a distal end (216) of the guide catheter, provides support for delivery of an interventional cardiology device in the form of a stent, a stent catheter, or a balloon catheter.

12. The guide extension catheter of claim 3, wherein the elongate tube member is biased to wrap around an outer surface of the guide catheter via two overlapping ends.

13. The guide extension catheter of any one of claims 1 to 12, wherein the length of the push member is less than the length of the guide catheter.

14. The guide extension catheter of any one of claims 1 to 13, wherein a difference between the cross-sectional inner diameter of the elongate tube member and a cross-sectional outer diameter of the guide catheter is about 0.051 mm (0.002 inches) to 0.0889 mm (0.0035 inches).

15. The guide extension catheter of any one of claims 1 to 14, wherein at least a distal portion of the elongate tube member is configured to reduce in diameter when advanced beyond a distal end of the guide catheter.

## Patentansprüche

1. Führungserweiterungskatheter (200) zur Verwendung mit einem Führungskatheter vordefinierter Länge (202), wobei der Führungserweiterungskatheter umfasst:
ein längliches Tubuselement (220), das ein Lumen (228) definiert, das einen Querschnittsinnendurchmesser aufweist, durch den der Führungskatheter einführbar ist; und
ein Vorschubelement (222), das ausreichend starr ist, um zu erlauben, dass das längliche Tubuselement über den Führungskatheter vorgeschoben wird, wobei das Vorschubelement proximal angeordnet ist, betrieblich damit verbunden ist und entlang einer Längsachse starrer ist als das längliche Tubuselement, wobei das Vorschubelement eine maximale Querschnittsabmessung aufweist, die kleiner ist als der äußere Querschnittsdurchmesser des länglichen Tubuselements, und eine Länge derart aufweist, dass es in Kombination mit der Länge des länglichen Tubuselements eine Führungserweiterungskatheter-Gesamtlänge entlang der Längsachse bildet, die länger ist als der Führungskatheter.

2. Führungserweiterungskatheter nach Anspruch 1, der weiter eine Einführschleuse (323) umfasst, die ein Lumen definiert, das einen Querschnittsinnendurchmesser aufweist, durch den das längliche Tubuselement einführbar ist.

3. Führungserweiterungskatheter nach einem der Ansprüche 1 oder 2, wobei das längliche Tubuselement einen Längsschlitz (829) entlang seiner Länge einschließt, wobei der Längsschlitz in Richtung einer geschlossenen Position vorgespannt ist.

4. Führungserweiterungskatheter nach einem der Ansprüche 1 bis 3, wobei der Innenquerschnittsdurchmesser des länglichen Tubuselements dazu konfiguriert ist, sich in Abhängigkeit von einer darauf angelegten Kraft einzustellen.

5. Führungserweiterungskatheter nach Anspruch 4, wobei das längliche Tubuselement ein Formgedächtnisgeflecht (333) umfasst.

6. Führungserweiterungskatheter nach Anspruch 5, wobei das Formgedächtnisgeflecht Nitinol umfasst.

7. Führungserweiterungskatheter nach Anspruch 4, wobei der Querschnittsinnendurchmesser des länglichen Tubuselements zum Abnehmen konfiguriert, bis der Querschnittsinnendurchmesser annähernd gleich einem Querschnittsaußendurchmesser des Führungskatheters ist.

8. Führungserweiterungskatheter nach einem der Ansprüche 1 bis 7, wobei das Vorschubelement einen Stab oder einen Draht umfasst.

9. Führungserweiterungskatheter nach einem der Ansprüche 1 bis 8, wobei das Vorschubelement eine Längsnut (744) entlang seiner Länge umfasst.

10. Führungserweiterungskatheter nach einem der Ansprüche 1 bis 9, wobei das längliche Tubuselement eine innere Polymerschicht und eine äußere Polymerschicht umfasst.

11. Führungserweiterungskatheter nach einem der Ansprüche 1 bis 10, wobei das längliche Tubuselement, wenn sein distales Ende distal über ein distales Ende (216) des Führungskatheters hinaus verlängert wird, Stützen zum Einbringen einer interventionellen kardiologischen Vorrichtung in der Form eines Stents, eines Stentkatheters oder eines Ballonkatheters bereitstellt.

12. Führungserweiterungskatheter nach Anspruch 3, wobei das längliche Tubuselement dazu vorgespannt ist, sich um eine Außenoberfläche des Führungskatheters über zwei einander überlappende Enden zu legen.

13. Führungserweiterungskatheter nach einem der Ansprüche 1 bis 12, wobei die Länge des Vorschubelements kleiner ist als die Länge des Führungskatheters.

14. Führungserweiterungskatheter nach einem der Ansprüche 1 bis 13, wobei ein Unterschied zwischen dem Innenquerschnittsdurchmesser des länglichen Tubuselements und dem Außenquerschnittsdurchmesser des Führungskatheters etwa 0,051 mm (0,002 Zoll) bis 0,0889 mm (0,0035 Zoll) beträgt.

15. Führungserweiterungskatheter nach einem der Ansprüche 1 bis 14, wobei mindestens ein distaler Abschnitt des länglichen Tubuselements dazu konfiguriert ist, den Durchmesser zu verringern, wenn er über das distale Ende des Führungskatheters hinaus vorgeschoben wird.

## Revendications

1. Cathéter d'extension de guidage (200) destiné à être utilisé avec un cathéter de guidage de longueur prédéfinie (202), le cathéter d'extension de guidage comprenant :
un élément tubulaire allongé (220) définissant une lumière (228) présentant un diamètre intérieur de section transversale à travers lequel le cathéter de guidage peut être inséré ; et
un élément de poussée (222) qui est suffisamment rigide pour permettre à l'élément tubulaire allongé d'être poussé sur le cathéter de guidage, l'élément de poussée étant proximal à, relié de manière fonctionnelle à et plus rigide le long d'un axe longitudinal que l'élément tubulaire allongé, l'élément de poussée présentant une dimension de section transversale maximale qui est inférieure à un diamètre extérieur de section transversale de l'élément tubulaire allongé et présentant une longueur telle que, lorsqu'elle est combinée à une longueur de l'élément tubulaire allongé, elle forme une longueur de cathéter d'extension de guidage le long de l'axe longitudinal qui est plus longue que le cathéter de guidage.

2. Cathéter d'extension de guidage selon la revendication 1, comprenant en outre une gaine d'introduction (323) définissant une lumière présentant un diamètre intérieur de section transversale à travers lequel l'élément tubulaire allongé peut être inséré.

3. Cathéter d'extension de guidage selon l'une quelconque des revendications 1 ou 2, dans lequel l'élément tubulaire allongé inclut une fente longitudinale (829) sur toute sa longueur, la fente longitudinale étant orientée vers une position fermée.

4. Cathéter d'extension de guidage selon l'une quelconque des revendications 1 à 3, dans lequel le diamètre intérieur de section transversale de l'élément tubulaire allongé est configuré pour s'ajuster en réponse à une force qui lui est appliquée.

5. Cathéter d'extension de guidage selon la revendication 4, dans lequel l'élément tubulaire allongé comprend une tresse à mémoire de forme (333).

6. Cathéter d'extension de guidage selon la revendication 5, dans lequel la tresse à mémoire de forme comprend du nitinol.

7. Cathéter d'extension de guidage selon la revendication 4, dans lequel le diamètre intérieur de section transversale de l'élément tubulaire allongé est configuré pour diminuer jusqu'à ce que le diamètre intérieur de section transversale soit approximativement égal au diamètre extérieur de section transversale du cathéter de guidage.

8. Cathéter d'extension de guidage selon l'une quelconque des revendications 1 à 7, dans lequel l'élément de poussée comprend une tige ou un fil.

9. Cathéter d'extension de guidage selon l'une quelconque des revendications 1 à 8, dans lequel l'élément de poussée comprend une rainure longitudinale (744) sur sa longueur.

10. Cathéter d'extension de guidage selon l'une quelconque des revendications 1 à 9, dans lequel l'élément tubulaire allongé comprend une couche polymère interne et une couche polymère externe.

11. Cathéter d'extension de guidage selon l'une quelconque des revendications 1 à 10, dans lequel l'élément tubulaire allongé, lorsque son extrémité distale est étendue distalement par rapport à une extrémité distale (216) du cathéter de guidage, fournit un support pour la livraison d'un dispositif de cardiologie interventionnelle sous la forme d'un stent, d'un cathéter stent ou d'un cathéter à ballonnet.

12. Cathéter d'extension de guidage selon la revendication 3, dans lequel l'élément tubulaire allongé est conçu pour s'enrouler autour d'une surface extérieure du cathéter de guidage via deux extrémités qui se chevauchent.

13. Cathéter d'extension de guidage selon l'une quelconque des revendications 1 à 12, dans lequel la longueur de l'élément de poussée est inférieure à la longueur du cathéter de guidage.

14. Cathéter d'extension de guidage selon l'une quelconque des revendications 1 à 13, dans lequel la différence entre le diamètre intérieur de section transversale de l'élément tubulaire allongé et le diamètre extérieur de section transversale du cathéter de guidage est d'environ 0,051 mm (0,002 pouces) à 0,0889 mm (0,0035 pouces).

15. Cathéter d'extension de guidage selon l'une quelconque des revendications 1 à 14, dans lequel au moins une partie distale de l'élément tubulaire allongé est configurée pour réduire son diamètre lorsqu'elle est avancée au-delà d'une extrémité distale du cathéter de guidage.
